# EUROPÄISCHE PATENTANMELDUNG

(11) **EP 3 332 747 A1**
(43) Veröffentlichungstag der Anmeldung: **13.06.2018**
(21) Anmeldenummer: 17205819.0
(22) Anmeldetag: 07.12.2017
(51) Int. Cl.: A61F 2/36

(54) **HÜFTGELENKENDOPROTHESENSYSTEM**

(30) Priorität: 09.12.2016 DE 102016123960
(71) Anmelder: Jooss, Armin, 73447 Oberkochen (DE)
(72) Erfinder: Jooß, Armin, 73447 Oberkochen (DE); Martin, Roland, 73432 Aalen-Oberkochen (DE)
(74) Vertreter: Frey, Sven Holger

(57) **Zusammenfassung**

Die Erfindung betrifft ein Hüftgelenkendoprothesensystem (2), wenigstens umfassend:
- eine Gelenkpfanne (4) zur Implantation in einem Beckenknochen (3) eines Patienten mit einem Gehäuse (4c) und einem Einsatz mit einer hohlkugeligen Kopfaufnahme,
- einen kugeligen Gelenkkopf (5), und
- eine Schaftprothese (6), welche einerseits mit dem Gelenkkopf (5) verbunden oder verbindbar ist und welche ferner wenigstens ein als perforierte Hülse ausgeführtes Rohrelement (8) aufweist, das in einem ersten Teilbereich (8a) zur Aufnahme eines Oberschenkelknochenteils (7) in einen Hohlraum (8b) des wenigstens einen Rohrelements (8) ausgelegt ist,
- wobei das wenigstens eine Rohrelement (8) in dem ersten Teilbereich (8a) mit einem oder mehreren Schraubenlöchern (9) zur Herstellung einer Schraubverbindung (10) zwischen dem wenigstens einen Rohrelement (8) und dem aufzunehmenden Oberschenkelknochenteil (7) versehen ist.

## Beschreibung

Die Erfindung betrifft ein Hüftgelenkendoprothesensystem, wenigstens umfassend eine Gelenkpfanne und einen Gelenkkopf.

Bekannte Hüftgelenkendoprothesen umfassen üblicher Weise eine Gelenkpfanne mit einem Pfanneneinsatz, welcher auch als Einsatz oder Inlay bezeichnet wird, einen in den Markkanal des Femurs implantierbaren Schaft und einen mit dem Schaft, insbesondere über einen Halsabschnitt, verbundenen oder verbindbaren Gelenkkopf. Der Pfanneneinsatz weist in der Regel eine Kopfaufnahme für den mit dem Femurschaft über den Halsabschnitt verbundenen oder verbindbaren kugeligen Gelenkkopf und einen umlaufenden Rand auf, wobei die Kopfaufnahme hohlkugelig geformt ist und einen ein Rotationszentrum definierenden Gelenkmittelpunkt definiert.

Ein derartiges Hüftgelenkendoprothesensystem ist in der DE 20 2012 100 571 U1 beschrieben.

Die Gelenkpfanne wird dabei häufig aus Stahl (legiert mit Chrom) mit einer Gleitbeschichtung aus Polyethylen oder Keramik gebildet. Die Gelenkpfanne kann ferner im Außenbereich für eine einwachsende, d. h. zementfreie Befestigung aufgeraut sein. Der Gelenkkopf kann aus legiertem Stahl oder Keramik gebildet und auf dem Schaft durch Presspassung oder mittels eines Gewindes am Schaftende befestigt sein. Der Schaft oder Prothesenschaft kann aus Stahl oder Titan gebildet sein und wird mit Hilfe von Knochenzement oder zementfrei mit aufgerauter Schaftoberfläche im Oberschenkelknochen bzw. Femur befestigt. Der Prothesenschaft kann eine konische Form aufweisen, welche an die Innenkontur des Knochens angepasst ist.

Bisherige Hüftgelenkendoprothesen weisen jedoch zahlreiche Nachteile und Risiken auf. Sie haben eine geringe Lebensdauer von 2 bis 10 Jahren - je nach Belastung und Körperverträglichkeit. Bei einem Austausch der Prothese ist ein komplizierter Eingriff und damit verbunden eine hohe Patientenbelastung erforderlich. Ein derartiger Austausch der Prothese (Schaft) ist nur maximal zweimal möglich und meist nur bei jüngeren Patienten, insbesondere mit Hilfe von Knochenzement. Des Weiteren sind die am Markt verfügbaren Hüftprothesen nur einsetzbar, wenn noch keine porösen Knochenstrukturen vorliegen, d. h. bei 30 bis 40 % der über 70-jährigen Patienten gibt es keine hinreichend sichere Lösung. Aufgrund des hohen Ausfallrisikos ist die Methode äußerst kostenintensiv und stellt keine zukunftssichere Lösung dar. Teilweise ergeben sich sehr geringe Lebensdauern aufgrund der Spaltwirkung des Prothesenschaftes auf den Knochen und einer damit einhergehenden möglichen Rissbildung im Knochen. Dies kann zu einer Lockerung des Schaftes mit Entzündungen und Schmerzen führen, welche einen Eingriff notwendig machen. Darüber hinaus können Allergien durch körperunverträgliche Materialien wie Kunststoffe oder Metallabrieb auftreten. Kobaltabrieb kann sogar toxische Reaktionen auslösen. Bei Patienten mit vorhandenen allergischen Empfindlichkeiten auf Kunststoffe oder Metallabrieb (auch im Nano-Bereich) ist keine Abhilfe mit hoher Sicherheit möglich. Darüber hinaus können ungleiche Beinlängen durch eine fehlende stabile Längenfixierung des Schaftes im Knochen entstehen. Es ist keine exakte Längenfixierung bereits bei der Operation möglich und bei einer Lockerung durch Knochenrisse besteht zudem die Gefahr eines Knochenbruchs.

Ausgehend davon liegt der vorliegenden Erfindung die Aufgabe zugrunde, ein Hüftgelenkendoprothesensystem der eingangs erwähnten Art zu verbessern, insbesondere die Lebensdauer des Hüftgelenkendoprothesensystems signifikant zu erhöhen und/oder eine exakte Längenfixierung bereits bei der Operation zu ermöglichen.

Diese Aufgabe wird erfindungsgemäß durch ein Hüftgelenkendoprothesensystem mit den in Anspruch 1 genannten Merkmalen gelöst.

Erfindungsgemäß wird ein Hüftgelenkendoprothesensystem vorgeschlagen, welches wenigstens umfasst:
- eine Gelenkpfanne, insbesondere zur Implantation in einem Beckenknochen eines Patienten, insbesondere mit einem Gehäuse und einem Einsatz mit einer hohlkugeligen Kopfaufnahme, und
- einen insbesondere kugeligen Gelenkkopf, sowie
- eine Schaftprothese, welche einerseits mit dem Gelenkkopf verbunden oder verbindbar ist und welche ferner wenigstens ein Rohrelement aufweist, das in einem ersten Teilbereich zur Aufnahme eines Oberschenkelknochenteils des Patienten in einem Hohlraum des wenigstens einen Rohrelements ausgelegt ist,
- wobei das wenigstens eine Rohrelement in dem ersten Teilbereich mit einem oder mehreren Schraubenlöchern zur Herstellung einer Schraubverbindung zwischen dem wenigstens einen Rohrelement und dem aufzunehmenden Oberschenkelknochenteil versehen ist.

Das erfindungsgemäße Hüftgelenkendoprothesensystem weist eine hohe Festigkeit bzw. eine hohe Lebensdauer auf. Dies ergibt sich einerseits durch einen geringen Verschleiß der Reibpaarung (Gelenkkopf und Gelenkpfannen-Einsatz aus Zirkonoxid) und andererseits durch den Einsatz des Rohrelements der Schaftprothese, welches als Hülse auf den Knochen gesetzt und verschraubt wird. Als Oberschenkelknochenteil kommt der Oberschenkelknochenschaft in Betracht. Hierzu kann der Oberschenkelknochen entsprechend gekürzt oder abgesägt, d. h. der Oberschenkelknochenkopf und der Oberschenkelknochenhals entfernt werden. Somit wird ein Sprengen bzw. eine Rissbildung beim Oberschenkelknochen - wie es bei herkömmlichen Hüftgelenkendoprothesen der Fall ist - wirksam vermieden. Des Weiteren besteht keine Allergiegefahr und somit eine hohe Verträglichkeit der Prothese, da kein kritischer Metallabrieb entsteht. Darüber hinaus kann eine exakte Längenfixierung bereits bei der Operation erfolgen und eine Variation in der Länge ist ebenfalls möglich. Somit könnte sogar ein verkürztes Bein wieder verlängert werden. Ungleiche Beinlängen werden durch eine stabile Längenfixierung mittels der Schaftprothese vermieden. Durch die einwachsende Schraubverbindung der Hülse bzw. des Rohrelements im Knochen wird eine sehr stabile und langlebige Anordnung geschaffen. Eine genaue Vermessung vorab gewährleistet eine exakte Anpassung des künstlichen Hüftgelenks auf den Patienten. Das Rohrelement ist sozusagen als perforierter Schaft ausgebildet.

Das Rohrelement ist als perforierte Hülse oder als perforierte Röhre ausgebildet. Die Geometrie des Rohrelements ist im Bereich des Knochenkontakts als perforierte Hülse oder als perforierte Röhre ausgeführt. Durch die Perforation wird ein natürliches Einwachsen von Knochenmasse (sogenannte Kallusbildung) in sehr vorteilhafter Weise begünstigt. Die Verwendung von schädlichem Knochenzement ist somit nicht erforderlich. Die Löcher der Perforation können als Schraubenlöcher zur Herstellung der Schraubverbindung zwischen dem wenigstens einen Rohrelement und dem aufzunehmenden Oberschenkelknochenteil verwendet werden.

Die Gelenkpfanne, die Schaftprothese und/oder das wenigstens eine Rohrelement der Schaftprothese können selbsteinwachsend ausgeführt sein. Dadurch lassen sich eine hohe Verträglichkeit und stabile Verbindungen erreichen.

Vorteilhaft ist es, wenn die Gelenkpfanne einen Einsatz oder ein Inlay bzw. einen Pfanneneinsatz insbesondere aus Zirkonoxid aufweist. Dadurch kann eine optimale Reibpaarung aus der Gelenkpfanne und dem Gelenkkopf gebildet werden. Die Verwendung von Zirkonoxid gewährleistet eine hohe Festigkeit und einen geringen Verschleiß.

Die Gelenkpfanne kann ein Gehäuse, insbesondere aus Chirurgenstahl oder Implantatstahl aufweisen.

Wenigstens eine Oberfläche der Gelenkpfanne oder des Gehäuses der Gelenkpfanne kann mit einer Struktur zur Verbesserung des Selbsteinwachsens versehen sein.

Vorteilhaft ist es, wenn der Gelenkkopf Zirkonoxid aufweist.

Dadurch lässt sich insbesondere in Verbindung mit einem Einsatz oder Inlay der Gelenkpfanne aus demselben Material eine beständige Reibpaarung mit geringstem Verschleiß schaffen. Hierdurch erhöht sich die Festigkeit bzw. Lebensdauer des gesamten Hüftgelenkendoprothesensystems.

Die Schaftprothese kann ferner wenigstens ein Knochennachbildungselement, insbesondere aus Zirkonoxid, aufweisen.

Als Teil eines Halsabschnittes oder als Halsabschnitt zwischen dem Gelenkkopf und dem Rohrelement der Schaftprothese kann ein Knochennachbildungselement vorhanden sein, welches dem Oberschenkelhals nachgebildet ist. Hierbei handelt es sich um einen Knochenersatz. Das Knochennachbildungselement kann zumindest teilweise aus Zirkonoxid gebildet sein. Das Knochennachbildungselement kann einteilig oder mehrteilig ausgeführt sein.

Das wenigstens eine Rohrelement kann aus Chirurgenstahl oder Implantatstahl gebildet und/oder 3D-formgefräst sein. Dadurch kann das Rohrelement bzw. die Schaftprothese sehr exakt auf den Patienten angepasst werden.

Das wenigstens eine Rohrelement kann einen zweiten, dem Gelenkkopf zugewandten oder zuzuwendenden Teilbereich aufweisen, in welchem das wenigstens eine Knochennachbildungselement befestigt ist. Zwischen dem ersten und dem zweiten Teilbereich kann sozusagen eine Trennstelle vorhanden sein, welche auch das Knochennachbildungselement von dem in dem ersten Teilbereich aufzunehmenden Oberschenkelknochenteil trennt.

Das wenigstens eine Knochennachbildungselement kann mittels Laserschweißen unverlierbar mit dem zweiten Teilbereich des wenigstens einen Rohrelements verbunden sein. Dadurch ergibt sich eine sehr stabile Befestigung.

Der Gelenkkopf kann mit dem wenigstens einen Knochennachbildungselement der Schaftprothese, lösbar verbunden, insbesondere verschraubt sein.

Das wenigstens eine Rohrelement oder der erste Teilbereich des wenigstens einen Rohrelements der Schaftprothese können in Form und/oder Größe an den Oberschenkelknochenteil, insbesondere den Oberschenkelknochenschaft eines Patienten angepasst sein.

Sehr vorteilhaft ist es, wenn der Gelenkkopf gegenüber der Schaftprothese verstellbar ist. Der Gelenkkopf kann somit relativ zu der Schaftprothese einstellbar fixierbar sein. Durch die lösbare Verbindung lässt sich die Kombination aus Gelenkkopf und Schaftprothese sowohl vor als auch nach dem Einbau bzw. der Operation exakt auf den Bedarfsfall einstellen. Dadurch erhält der Gelenkkopf die optimale Lage und Vorspannung in der Gelenkpfanne für eine sichere Funktion.

Der Gelenkkopf kann mit einem Schaft versehen sein, welcher insbesondere einen abgeflachten Bereich bzw. eine Anflachung aufweist. Bei der Operationsvorbereitung kann durch Austausch aus unterschiedlichen Varianten von Gelenkkopf-Schaft-Modulen die optimale Länge ausgewählt werden. Sollte beispielsweise aufgrund eines Unfalls das Gelenk beschädigt werden, so kann ohne hohen Aufwand das vorhandene Gelenkkopf-Schaft-Modul gelöst und ein neues Teil eingebaut werden.

Die Schaftprothese kann eine erste, insbesondere kanalartige Ausnehmung bzw. einen ersten Kanal zur Aufnahme des Schaftes des Gelenkkopfs umfassen.

Der Schaft des Gelenkkopfs kann in der ersten Ausnehmung der Schaftprothese, insbesondere mittels eines Ziehkeils bzw. einer Ziehkeileinrichtung lösbar fixierbar sein. Die Ziehkeileinrichtung kann in einer zweiten, insbesondere kanalartigen Ausnehmung bzw. einem zweiten Kanal der Schaftprothese angeordnet sein, welche/welcher im Bereich der ersten Ausnehmung im Wesentlichen senkrecht zu dieser verläuft. Die erste und/oder zweite, insbesondere kanalartige Ausnehmung kann auch eine Bohrung sein. Die erste und die zweite Ausnehmung können miteinander verbunden sein, um einen Eingriff bzw. einen Kontakt der Ziehkeileinrichtung, insbesondere mit dem abgeflachten Bereich des Schafts des Gelenkkopfs zu ermöglichen.

Das Rohrelement kann mehrteilig, insbesondere zweiteilig ausgeführt sein. Die einzelnen Teile können z. B. über Schraubverbindungen zusammengefügt werden.

Vorteilhafte Ausgestaltungen und Weiterbildungen der Erfindung ergeben sich aus den Unteransprüchen.

Nachfolgend ist anhand der Zeichnung prinzipmäßig ein Ausführungsbeispiel der Erfindung angegeben.

Es zeigen:
- Fig. 1: eine schematische Darstellung eines implantierten, erfindungsgemäßen Hüftgelenkendoprothesensystems;
- Fig. 2: eine schematische Darstellung einer ersten Ausführungsform eines implantierten, erfindungsgemäßen Hüftgelenkendoprothesensystems;
- Fig. 3: eine schematische Darstellung einer zweiten Ausführungsform eines implantierten, erfindungsgemäßen Hüftgelenkendoprothesensystems;
- Fig. 4: eine schematische Darstellung eines Ausschnitts x des in Fig. 2 gezeigten Hüftgelenkendoprothesensystems; und
- Fig. 5: eine schematische Darstellung des Schnittes A-B aus Fig. 4.

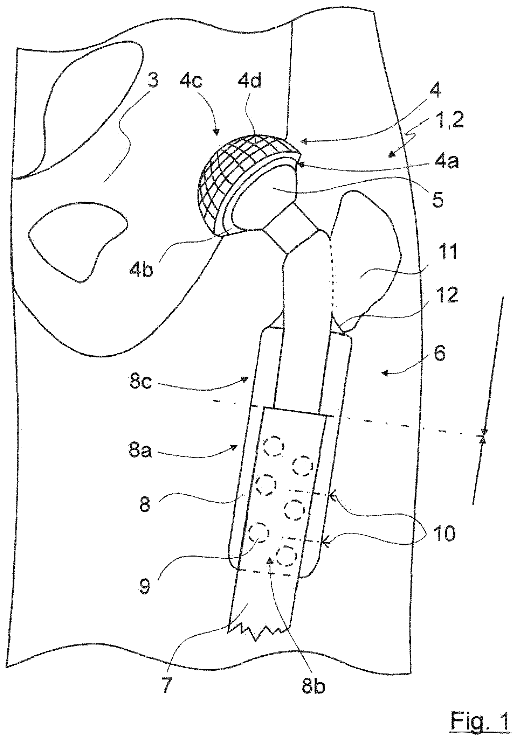

In Figur 1 ist schematisch eine Hüftgelenkendoprothese 1 eines erfindungsgemäßen Hüftgelenkendoprothesensystems 2 dargestellt. Das Hüftgelenkendoprothesensystem 2 umfasst eine in einen Beckenknochen 3 eines Patienten implantierbare Gelenkpfanne 4 mit einem Einsatz 4a, welcher auch als Pfanneneinsatz oder Inlay bezeichnet wird. Der Einsatz 4a ist in Form einer Kugelschale mit einer nicht näher dargestellten hohlkugeligen Kopfaufnahme ausgebildet. Begrenzt wird die Kopfaufnahme von einem umlaufenden Rand 4b des Einsatzes 4a. Ein kugeliger Gelenkkopf 5 bildet zusammen mit dem Einsatz 4a der Gelenkpfanne 4 ein Kugelgelenk. Der Gelenkkopf 5 ist über eine Schaftprothese 6 mit einem als Oberschenkelknochenschaft (Femurschaft) ausgebildeten Oberschenkelknochenteil 7 verbunden. Die Schaftprothese 6, welche einerseits mit dem Gelenkkopf 5 verbunden oder verbindbar ist, weist ferner ein Rohrelement 8 auf, das in einem ersten Teilbereich 8a zur Aufnahme des Oberschenkelknochenteils 7 in einem Hohlraum 8b des wenigstens einen Rohrelements 8 ausgelegt ist. Das Rohrelement 8 ist in dem ersten Teilbereich 8a mit mehreren Schraubenlöchern 9 zur Herstellung einer Schraubverbindung 10 zwischen dem Rohrelement 8 und dem aufzunehmenden Oberschenkelteil 7 versehen. Das Rohrelement 8 ist somit als perforierter Schaft bzw. als perforierte Hülse ausgebildet.

Wie aus Figur 1 ersichtlich, ist bei dem Oberschenkelknochen der Oberschenkelknochenkopf und der Oberschenkelknochenhals entfernt worden.

Die Gelenkpfanne 4, die Schaftprothese 6 und/oder das wenigstens eine Rohrelement 8 der Schaftprothese 6 können selbsteinwachsend ausgeführt sein.

Der Einsatz 4a der Gelenkpfanne 4 kann aus Zirkonoxid gebildet sein.

Wie aus der Figur ersichtlich, weist die Gelenkpfanne 4 ein Gehäuse 4c auf, welches aus Chirurgenstahl oder Implantatstahl gebildet ist.

Eine Oberfläche der Gelenkpfanne 4 oder des Gehäuses 4c der Gelenkpfanne 4 ist mit einer Struktur 4d zur Verbesserung des Selbsteinwachsens versehen.

Im vorliegenden Ausführungsbeispiel weist der Gelenkkopf 5 Zirkonoxid auf.

Wie aus Figur 1 ersichtlich, weist die Schaftprothese 6 ferner ein Knochennachbildungselement 11, insbesondere aus Zirkonoxid, auf. Das Knochennachbildungselement 11 kann auch mehrteilig ausgeführt sein (in Figur 1 durch eine gestrichelte Linie angedeutet) bzw. es können auch mehrere Knochennachbildungselemente vorhanden sein.

Das Rohrelement 8 kann aus Chirurgenstahl oder Implantatstahl gebildet sein. Im vorliegenden Ausführungsbeispiel ist das Rohrelement 8 3D-formgefräst.

Wie aus Figur 1 ersichtlich, weist das Rohrelement 8 einen zweiten, dem Gelenkkopf 5 zugewandten oder zuzuwendenden Teilbereich 8c auf, in welchem das Knochennachbildungselement 11 befestigt oder aufgenommen ist. Eine Trennstelle zwischen dem ersten Teilbereich 8a und dem zweiten Teilbereich 8c des Rohrelements 8 ist in Figur 1 durch Pfeile und eine strichpunktierte Linie angedeutet.

Das Knochennachbildungselement 11 ist mittels Laserschweißen unverlierbar mit dem zweiten Teilbereich 8c des Rohrelements 8 verbunden (durch das Bezugszeichen 12 angedeutet).

Der Gelenkkopf 5 ist mit dem Knochennachbildungselement 11 der Schaftprothese 6 lösbar verbunden, insbesondere verschraubt.

Das Rohrelement 8 oder der erste Teilbereich 8a des Rohrelements 8 der Schaftprothese 6 ist in Form und/oder Größe an den Oberschenkelknochenteil 7, insbesondere den Oberschenkelknochenschaft angepasst.

In den Figuren sind funktionsgleiche Elemente mit denselben Bezugszeichen versehen.

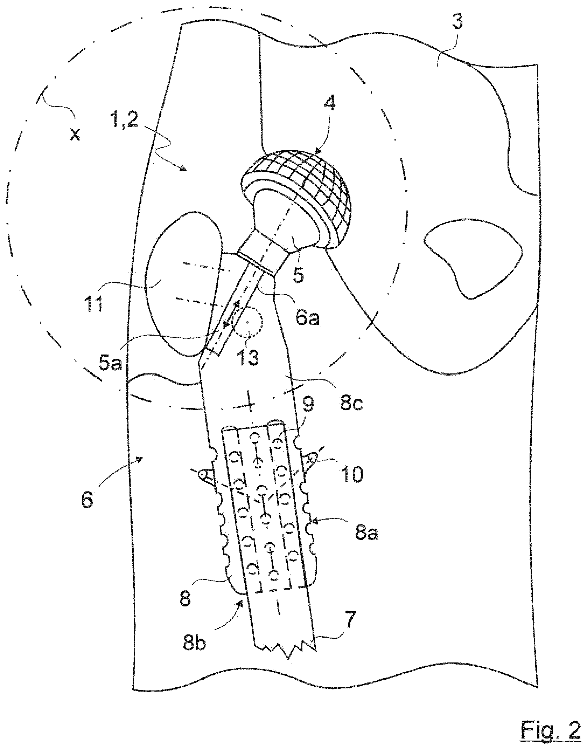

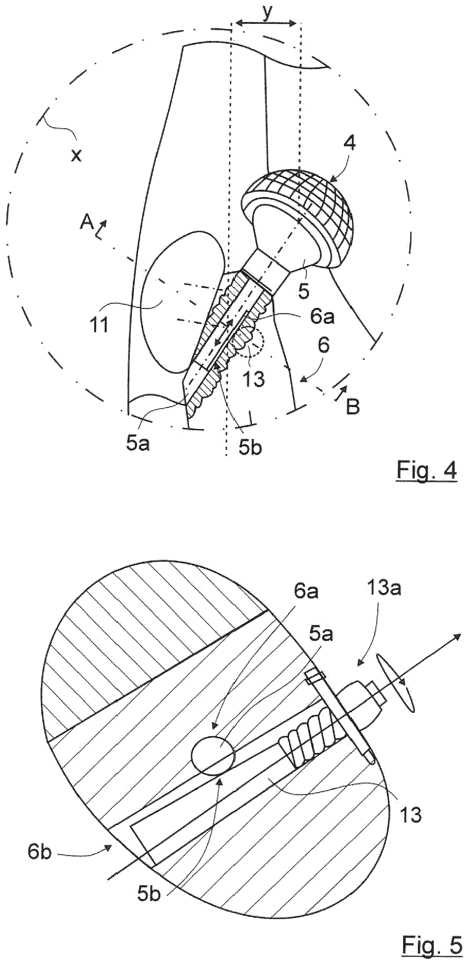

In Figur 2 ist das erfindungsgemäße Hüftgelenkendoprothesensystem 2 in einer ersten Ausführungsform gezeigt. Ein Teilbereich x der Figur 2 ist in Figur 4 näher gezeigt.

Wie aus Figur 2 ersichtlich, ist der Gelenkkopf 5 gegenüber der Schaftprothese 6 verstellbar ausgeführt. Der Gelenkkopf 5 ist mit einem Schaft 5a versehen, welcher einen abgeflachten Bereich 5b (siehe Figur 4) aufweist. Die Schaftprothese 6 umfasst eine erste Ausnehmung 6a zur Aufnahme des Schaftes 5a des Gelenkkopfs 5. Der Schaft 5a des Gelenkkopfs 5 ist in der ersten Ausnehmung 6a der Schaftprothese 6, insbesondere mittels eines Ziehkeils oder Ziehkeileinrichtung 13 lösbar fixierbar bzw. lösbar fixiert und dadurch ausziehbar aus der Schaftprothese 6 gestaltet. Die Ziehkeileinrichtung 13 kann in einer zweiten Ausnehmung 6b der Schaftprothese 6 angeordnet sein, welche im Bereich der ersten Ausnehmung 6a im Wesentlichen senkrecht zu dieser verläuft (siehe Figur 5). Die erste und die zweite Ausnehmung 6a, 6b können miteinander verbunden sein, um einen Eingriff bzw. einen Kontakt der Ziehkeileinrichtung 13, insbesondere mit dem abgeflachten Bereich 5b des Schafts 5a des Gelenkkopfs 5 zu ermöglichen (siehe Figur 5), wodurch eine Befestigung des Schafts 5a durch dessen Blockierung mittels der Ziehkieleinrichtung 13 erzielt werden kann.

Das Rohrelement 8 ist als perforierter Schaft bzw. als perforierte Hülse oder Röhre ausgebildet. Die Perforation wird durch die Schraubenlöcher 9 gebildet bzw. die Löcher der Perforation können auch für die Schraubverbindungen 10 zum Oberschenkelknochenteil 7 verwendet werden. In Figur 2 sind zwei Schraubverbindungen 10 dargestellt. Es können mehrere, beispielsweise zwei bis vier Schraubverbindungen 10 verwendet werden. Die Axialbefestigung des Oberschenkelknochenteils 7 in dem Rohrelement 8 kann mittels selbstschneidender Schrauben als Schraubverbindungen 10 erfolgen (nicht näher dargestellt).

Im vorliegenden Ausführungsbeispiel verläuft das Rohrelement 8 bis zum Gelenkkopf 5 bzw. nimmt dessen Schaft 5a auf.

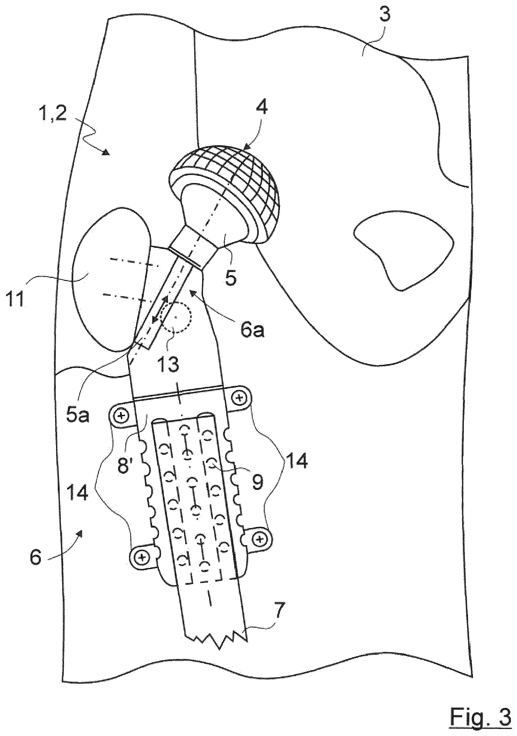

Die in Figur 3 dargestellte zweite Ausführungsform des erfindungsgemäße Hüftgelenkendoprothesensystems 2 weist ein Rohrelement 8' auf, welches mehrteilig, vorliegend zweiteilig ausgeführt ist. Das Rohrelement 8' ist sozusagen teilbar. Es kann z. B. zwei Halbschalen aufweisen, welche über Schraubverbindungen 14 miteinander verbunden sind (in Figur 3 vereinfacht dargestellt).

In Figur 4 ist der Ausschnitt x der Figur 2 näher dargestellt. Dabei ist der abgeflachte Bereich 5b des Schaftes 5a des Gelenkkopfs 5 ersichtlich.

Figur 5 zeigt den Schnitt A-B aus Figur 4. Dabei weist die Ziehkeileinrichtung 13 bzw. der Ziehkeil eine selbstsichernde Mutter 13a auf. In Figur 5 ist die Ziehkeileinrichtung 13 in einer blockierenden Stellung gezeigt, wobei der Schaft 5a fixiert ist. Der abgeflachte Bereich 5b des Schafts 5a steht mit der Ziehkeileinrichtung 13 in Wirkverbindung. Durch eine Rechtsdrehung der Mutter 13a gleitet die Ziehkeileinrichtung 13 nach außen bis zu dem Kontakt mit dem abgeflachten Bereich 5b des Schafts 5a. Es kommt zu einer festen Verbindung bei gleichzeitiger Verdrehsicherung des Schafts 5a mit dem Gelenkkopf 5. Durch das Lösen der Mutter 13a kann der Schaft 5a neu positioniert und fixiert werden. Durch die lösbare Verbindung mittels der Ziehkeileinrichtung 13 lassen sich Gelenkkopf 5 und Schaft 5a sowohl vor als auch nach dem Einbau exakt auf den Bedarfsfall einstellen (durch ein Maß y in Figur 5 angedeutet). Dadurch erhält der Gelenkkopf 5 eine optimale Lage und Vorspannung in der Gelenkpfanne 4 für eine sichere Funktion des Gelenks.

Das erfindungsgemäße Hüftgelenk-Implantat weist einen modularen Aufbau für höchste Lebensdauer und Bioverträglichkeit auf. Das Rohrelement 8, 8' bzw. die perforierte Hülse bildet ein erstes Modul. Der Gelenkkopf 5 und dessen Schaft 5a bilden ein zweites Modul. Ein drittes Modul umfasst die Gelenkpfanne 4 mit deren Pfannenkappe bzw. Gehäuse 4c. Bei der Operationsvorbereitung kann man durch verschiedene Varianten des zweiten Moduls die optimale Länge durch Austausch auswählen. Sollte z. B. aufgrund eines Unfalls das Gelenk beschädigt werden, so kann man ohne großen Aufwand das zweite Modul lösen und ein Ersatzteil einbauen.

### Bezugszeichenliste

- 1: Hüftgelenkendoprothese
- 2: Hüftgelenkendoprothesensystem
- 3: Beckenknochen
- 4: Gelenkpfanne
- 4a: Einsatz
- 4b: umlaufender Rand
- 4c: Gehäuse der Gelenkpfanne
- 4d: Struktur
- 5: Gelenkkopf
- 5a: Schaft des Gelenkkopfs
- 5b: abgeflachter Bereich
- 6: Schaftprothese
- 6a: erste Ausnehmung der Schaftprothese
- 6b: zweite Ausnehmung der Schaftprothese
- 7: Oberschenkelknochenteil
- 8, 8': Rohrelement
- 8a: erster Teilbereich des Rohrelements
- 8b: Hohlraum des Rohrelements
- 8c: zweiter Teilbereich des Rohrelements
- 9: Schraubenlöcher
- 10: Schraubverbindung
- 11: Knochennachbildungselement
- 12: Laserschweißbereiche
- 13: Ziehkeileinrichtung
- 13a: Mutter
- 14: Schraubverbindung

## Patentansprüche

1. Hüftgelenkendoprothesensystem (2), wenigstens umfassend:
- eine Gelenkpfanne (4) zur Implantation in einem Beckenknochen (3) eines Patienten mit einem Gehäuse (4c) und einem Einsatz mit einer hohlkugeligen Kopfaufnahme, und
- einen kugeligen Gelenkkopf (5),
**gekennzeichnet durch**
- eine Schaftprothese (6), welche einerseits mit dem Gelenkkopf (5) verbunden oder verbindbar ist und welche ferner wenigstens ein als perforierte Hülse ausgeführtes Rohrelement (8,8') aufweist, das in einem ersten Teilbereich (8a) zur Aufnahme eines Oberschenkelknochenteils (7) in einen Hohlraum (8b) des wenigstens einen Rohrelements (8) ausgelegt ist,
- wobei das wenigstens eine Rohrelement (8,8') in dem ersten Teilbereich (8a) mit einem oder mehreren Schraubenlöchern (9) zur Herstellung einer Schraubverbindung (10) zwischen dem wenigstens einen Rohrelement (8,8') und dem aufzunehmenden Oberschenkelknochenteil (7) versehen ist.

2. Hüftgelenkendoprothesensystem (2) nach Anspruch 1, wobei die Gelenkpfanne (4), die Schaftprothese (6) und/oder das wenigstens eine Rohrelement (8,8') der Schaftprothese (6) selbsteinwachsend ausgeführt sind.

3. Hüftgelenkendoprothesensystem (2) nach Anspruch 1 oder 2, wobei die Gelenkpfanne (4) einen Einsatz (4a), insbesondere aus Zirkonoxid, aufweist

4. Hüftgelenkendoprothesensystem (2) nach Anspruch 1, 2 oder 3, wobei die Gelenkpfanne (4) ein Gehäuse (4c), insbesondere aus Chirurgenstahl oder Implantatstahl, aufweist.

5. Hüftgelenkendoprothesensystem (2) nach einem der Ansprüche 1 bis 4, wobei wenigstens eine Oberfläche der Gelenkpfanne (4) oder des Gehäuses (4c) der Gelenkpfanne (4) mit einer Struktur (4d) zur Verbesserung des Selbsteinwachsens versehen ist.

6. Hüftgelenkendoprothesensystem (2) nach einem der Ansprüche 1 bis 5, wobei die Schaftprothese (6) ferner wenigstens ein Knochennachbildungselement (11), insbesondere aus Zirkonoxid, aufweist.

7. Hüftgelenkendoprothesensystem (2) nach einem der Ansprüche 1 bis 6, wobei das wenigstens eine Rohrelement (8,8') aus Chirurgenstahl oder Implantatstahl gebildet und/oder 3D-formgefräst ist.

8. Hüftgelenkendoprothesensystem (2) nach Anspruch 6 oder 7, wobei das wenigstens eine Rohrelement (8,8') einen zweiten, dem Gelenkkopf (5) zugewandten oder zuzuwendenden Teilbereich (8c) aufweist, in welchem das wenigstens eine Knochennachbildungselement (11) befestigt ist.

9. Hüftgelenkendoprothesensystem (2) nach Anspruch 8, wobei das wenigstens eine Knochennachbildungselement (11) mittels Laserschweißen unverlierbar mit dem zweiten Teilbereich (8c) des wenigstens einen Rohrelements (8,8') verbunden ist.

10. Hüftgelenkendoprothesensystem (2) nach einem der Ansprüche 6 bis 9, wobei der Gelenkkopf (5) mit dem wenigstens einen Knochennachbildungselement (11) der Schaftprothese (6) lösbar verbunden, insbesondere verschraubt ist.

11. Hüftgelenkendoprothesensystem (2) nach einem der Ansprüche 1 bis 10, wobei das wenigstens eine Rohrelement (8,8') oder der erste Teilbereich (8a) des wenigstens einen Rohrelements (8,8') der Schaftprothese (6) in Form und/oder Größe an den Oberschenkelknochenteil (7), insbesondere an den Oberschenkelknochenschaft angepasst ist.

12. Hüftgelenkendoprothesensystem (2) nach einem der Ansprüche 1 bis 11, wobei der Gelenkkopf (5) gegenüber der Schaftprothese (6) verstellbar ist.

13. Hüftgelenkendoprothesensystem (2) nach einem der Ansprüche 1 bis 12, wobei der Gelenkkopf (5) mit einem Schaft (5a) versehen ist, welcher einen abgeflachten Bereich (5b) aufweist.

14. Hüftgelenkendoprothesensystem (2) nach Anspruch 13, wobei die Schaftprothese (6) eine erste Ausnehmung (6a) zur Aufnahme des Schaftes (5a) des Gelenkkopfs (5) umfasst.

15. Hüftgelenkendoprothesensystem (2) nach Anspruch 14, wobei der Schaft (5a) des Gelenkkopfs (5) in der ersten Ausnehmung (6a) der Schaftprothese (6), insbesondere mittels einer Ziehkeileinrichtung (13) lösbar fixierbar ist.

16. Hüftgelenkendoprothesensystem (2) nach Anspruch 15, wobei die Ziehkeileinrichtung (13) in einer zweiten Ausnehmung (6b) der Schaftprothese (6) angeordnet ist, welche im Bereich der ersten Ausnehmung (6a) im Wesentlichen senkrecht zu dieser verläuft.

17. Hüftgelenkendoprothesensystem (2) nach einem der Ansprüche 1 bis 16, wobei das Rohrelement (8') mehrteilig, insbesondere zweiteilig ausgeführt ist.
